Europäisches Patentamt

European Patent Office

Office européen des brevets

(11) Veröffentlichungsnummer: **0 498 147 A1**

## EUROPÄISCHE PATENTANMELDUNG

(21) Anmeldenummer: **92100038.6**

(51) Int. Cl.⁵: **B09B 3/00**

(22) Anmeldetag: **03.01.92**

(30) Priorität: **07.02.91 IE 412/91**

(43) Veröffentlichungstag der Anmeldung:
**12.08.92 Patentblatt 92/33**

(84) Benannte Vertragsstaaten:
**AT BE CH DE DK ES FR GB GR IT LI LU MC NL PT SE**

(71) Anmelder: **H.P. Chemie Pelzer Research & Development Ltd.**
**Villa Thomas, Doneraile Walk**
**Tramore, County Waterford(IE)**

(72) Erfinder: **Ebner, Hans Georg, Dr.**
**Mergelweg 11**
**W-4300 Essen 14(DE)**
Erfinder: **Knackstedt, Hans-Günther**
**Wittener Bruch 6**
**W-5810 Witten(DE)**
Erfinder: **Sprenger, Bertold, Dr.**
**Portmannsweg 45**
**W-4600 Dortmund 72(DE)**

(74) Vertreter: **Schneider, Wilhelm, Dipl.-Phys.**
**Merckelbachweg 3**
**W-4770 Soest(DE)**

(54) **Flachbett-Reaktor zur biologischen Reinigung von mit gesundheitsschädlichen Stoffen verunreinigten Feststoffen und Verfahren zum Reinigen mit solchem Flachbett-Reaktor.**

(57) Die Erfindung betrifft einen Flachbett-Reaktor zur biologischen Reinigung von mit gesundheitsschädlichen Stoffen, insbesondere Kohlenwasserstoffen verunreinigten Böden z. B. mit hohem Ton- und Schluffgehalt durch Bakterienkulturen.

Die erfinderische Lösung besteht darin, daß die verunreinigten Feststoffe mit Bakterienkulturen und wässeriger Nährsalzlösung in einem horizontalen, transportablen Flachbett-Reaktor behandelt werden, der aus einer nach oben offenen, rechteckigen Wanne (1) mit Klapptür (2) und siebartig gelochtem Boden (3) besteht, sowie mit Belüftungs- (4) und Mischeinrichtung (5) und einem Belüftungssystem (6) ausgerüstet ist.

Die Wände der Wanne (1) sind als stapelbares und kippbares Tragegerüst (7) mit Halterungen (8) ausgestattet. Über eine Bodenwanne (16) können mehrere Flachbett-Reaktoren übereinander gestapelt sein.

Die Erfindung betrifft einen Flachbett-Reaktor zur biologischen Reinigung von mit gesundheitsschädlichen Stoffen, insbesondere Kohlenwasserstoffen verunreinigten Feststoffen z.B.mit hohem Ton- und Schluffgehalt durch Bakterienkulturen und wässerige Nährlösungen.

Das Problem der Reinigung kontaminierter Böden, hat zur Schaffung verschiedener Verfahren und Vorrichtungen geführt, von denen beispielsweise die Deponierung, die Verbrennung, die biologische in-situ-Behandlung, die biologische Behandlung im Beet sowie chemische Wasch- und Extraktionsverfahren zu erwähnen sind.

Es ist bekannt, die Dekontaminierung von verunreinigten Böden durch verschiedene Varianten von Extraktionsverfahren und entsprechenden Vorrichtungen zu erreichen. Die meisten dieser technischen Lösungen sind sehr apparate- und kostenaufwendige "Durchlaufprozesse", die zudem aufgrund ihrer Abmaße noch mit Transportproblemen behaftet sind.

Weitere Extraktionsverfahren arbeiten nach dem Prinzip eines Betonmischers aufgebauten Extraktionsmischers,dem in Abhängigkeit vom Kontaminierungsgrad mehrere Waschtrommeln nachgeordnet sind. Die verunreinigten Böden werden in mehreren Stufen hintereinander mit einem Extrakionsmittel bzw. Wasser gemischt und das in jeder Stufe anfallende Lösungsgemisch mit seinem jeweiligen Anteil an Schadstoffkonzentrat von den Feststoffen getrennt und abgezogen. Nachteilig bei diesen Varianten ist, daß diese Lösungen sehr unflexibel sind (DE-OS 37 26 282).

Bei einigen biologischen Reinigungsverfahren werden Drehtrommeln eingesetzt. So werden gemäß DE-OS 38 24 009 aus der kontaminierten Erde unter Zugabe von Extrakionsflüssigkeit in der Drehtrommel Kugeln geformt, aus denen mittels Preßwalzen die Schadflüssigkeit ausgepreßt wird. Die Kugeln werden anschließend wieder zerkleinert. Dem zerkleinerten Boden können dann Mikroorganismen zugesetzt werden.

Die Gegenstrom-Extraktion ist mit dem Nachteil behaftet, daß die Durchmischung von Lösung und zu reinigenden Böden sowie die Verweilzeit dieser Stoffe im Gegenstrom-Extraktor zu kurz sind. Demzufolge entspricht der Reinigungsgrad der behandelten Böden weder den Erwartungen noch den amtlichen Forderungen, die an den Reinigungsgrad gestellt werden.

Eine weitere Methode zur Dekontaminierung von Böden ist die thermische Behandlung in Wärmebehandlungsapparaten wie Drehrohröfen oder Pyrolysekammern. Diese Verfahren müssen jedoch mit einem hohen Energiebedarf und extrem hohen Betriebskosten betrieben werden (DE-OS 37 06 684 und DE-PS 37 25 301).

All diese Verfahren sind mit spezifischen Nachteilen behaftet. Sie erfordern einen erheblichen apparatetechnischen Aufwand und sind unflexibel. Außerdem führen sie teilweise zu Umweltbelastungen, zu beträchlichen Mengen an kritischem Deponiegut oder erfordern extrem hohe Betriebskosten, bei denen der gereinigte Boden danach keine organischen Bestandteile mehr aufweist und damit biologisch tot ist (Behandlung in Drehrohröfen und Pyrolysekammern).

Die Erfindung liegt die Aufgabe zugrunde, einen horizontalen, transportablen Flachbett-Reaktor zur biologischen Reinigung von mit gesundheitsschädlichen Feststoffen, insbesondere Kohlenwasserstoffen verunreinigten Böden z.B.mit hohem Ton-Schluff- und Flüssigkeitsgehalt durch Bakterienkulturen und wässerige Nährlösungen zu entwickeln, der mehrfach übereinander stapelbar ist, sowie über solche Abmaße verfügt,daß er ohne Sondergenehmigung auf Straßen und Schienen transportiert und ohne zusätzliche Beladungseinrichtungen wie beispielsweise Gabelstapler oder Kran auf Lastkraftwagen verladen werden kann.

Erfindungsgemäß wird die Aufgabe dadurch gelöst, daß der Flachbett-Reaktor aus einer nach oben offenen, rechteckigen Wanne mit Klapptür und siebartig gelochtem Boden besteht, sowie mit Belüftungs- und - je nach Bodenart - mit einer optionalen Mischeinrichtung und einem Berieselungssystem ausgerüstet ist.

Eine Wand der Wanne ist als abgedichtete Klapptür gestaltet und der Boden ist zur Vermeidung des Verlustes von Feststoff beim Transport durch den siebartig gelochten Boden mit einer Filterschicht aus verrottbarem organischen Material abgedeckt.

Die Wände der Wanne sind als Tragegerüst mit Halterungen ausgestaltet, um mehrere Flachbett-Reaktoren über eine Bodenwanne stapeln und zum Entleeren kippen zu können.

Die Ecken der Wanne sind zum Verladen des Flachbett-Reaktors mittels Seilgehänge mit kranfähigen Anschlagösen versehen.

Die Belüftungseinrichtung besteht aus in Längsrichtung an den Innenwänden und am Boden der Wanne fest angebrachten, perforierten Belüftungsrohren, die am gegenüberliegenden Ende zur Stirnseite mit abnehmbaren Kappen verschlossen sind die zu Reinigungszwecken abgenommen werden können. Zur Versorgung der Mikroorganismen mit Sauerstoff wird der Flachbett-Reaktor stirnseitig über die perforierten Belüftungsrohre mit Luft beaufschlagt.

Die Mischungseinrichtungen für die Durchmischung des verunreinigten Bodens mit hohem Ton- und Schluffgehalt mit den Mikroorganismen und der wässerigen Nährsalzzugabe besteht aus mehreren, vorzugsweise drei bis vier achsparallel angeordneten Mischwellen mit aufgesetzten Rührpaddeln und ist mit einem hydraulischen, elektrischen oder externen Antrieb ausgestattet.

Der Flachbett-Reaktor ist zur Bewässerung und Nährsalzzugabe mit einem Berieselungssystem ausgerüstet.

Der erfindungsgemäße Flachbett-Reaktor verfügt über ein Bruttovolumen von max. 16 m hoch 3 und erreicht damit ein Gesammtgewicht von ca. 30 Mg. Damit weist der Flachbett-Reaktor solche Abmaße auf, daß er ohne Sondergenehmigung für den Straßen- und Schienentransport zugelassen ist. Für den Straßentransport werden die Einrichtungen von selbstladenden Fahrzeugen benutzt, so daß nicht auf zusätzliche Beladungseinrichtungen wie Gabelstapler oder Kran zurückgegriffen werden muß.

Der erfindungsgemäße Flachbett-Reaktor zur biologischen Reinigung von mit gesundheitsschädlichen Stoffen, insbesondere Kohlenwasserstoffen verunreinigten Böden wird nachfolgend anhand einer Zeichnung näher erläutert.

Der mit Kohlenwasserstoffen verunreinigte Boden,der einen Flüssigkeitsgehalt von bis zu 40% aufweisen kann, gelangt vor Ort über einen Radlader oder Bagger in den Flachbett-Reaktor, der aus einer nach oben offenen, rechteckigen Wanne (1) mit Klapptür (2) und siebartig gelochtem Boden (3) besteht, sowie mit Belüftungs- (4) und Mischeinrichtungen (5) und einem Berieselungssystem (6) ausgerüstet ist. Der Boden (3) der Wanne (1) ist zur Vermeidung des Verlustes von Feststoff beim Transport durch den siebartig gelochten Boden (3) mit einer Filterschicht aus später verrottbarem organischen Material, beispielsweise Filterpapier bedeckt.

Zur Versorgung der Mikroorganismen mit Sauerstoff wird der Flachbett-Reaktor stirnseitig über die Belüftungseinrichtung (4), deren perforierten Belüftungsrohre (12) an den Innenwänden (11) der Wanne (1) fest angebracht sind, mit einer variablen Menge Luft beaufschlagt. Die am gegenüberliegenden Ende der Belüftungsrohre (12) aufgesetzten Kappen (13) werden zu Reinigungszwecken abgenommen.

Die Bewässerung und Nährsalzversorgung des verunreinigten Bodens erfolgt über ein externes Berieselungssystem (6).

Durch die Mischungseinrichtung (5), die vorzugsweise aus drei bis vier achsparallel angeordneten Mischerwellen (14) mit aufgesezten Rührpaddeln (15) besteht, wird der verunreinigte Boden, insbesondere bei hohem Ton- und Schluffgehalt, mit Mikroorganismen, einer variablen Menge Nährsalzlösung und Wasser intensiv gemischt.Der Antrieb der langsam drehenden Mischerwellen (14) erfolgt beispielsweise elektrisch.

Zur effektiveren, Mehrfachtapelung der Flachbett-Reaktoren sind die Wände der Wanne (1) als Tragegerüst (7) mit Halterungen (8) ausgestattet.

Die Flachbett-Reaktoren werden bei Temperaturen von ca. 25°C betrieben. Die Entwässerung erfolgt durch Durchsickern des Wassers und der Nährsalzlösung durch die mit einer Filterschicht bedeckten siebartig gelochten Böden (3) der gestapelten Flachbett-Reaktoren bis zur Bodenwanne (16).

Die Verweilzeit in den Flachbett-Reaktoren kann bis zu mehreren Monaten betragen.

Die Entladung des Flachbett-Reaktors erfolgt durch Kippen und Öffnen der Klapptür (2), die zur Vermeidung von Flüssigkeitsaustritt mit einer Dichtung (10) ausgerüstet ist, da durch Vibration beim Transport Entmischungsvorgänge auftreten können.

Weiterer Gegenstand der Erfindung ist ein Verfahren zur biologischen Reinigung von Böden mit einem Flachbett-Reaktor gemäß dieser Erfindung. Das Verfahren besteht darin, daß die maximale Masse der eingebrachten Feststoffe 28 Mg beträgt und bei einer Betriebstemperatur von 25° C je nach Kontaminierungsgrad des Bodens in dem Reaktor bis zu einer Verweilzeit von mehreren Monaten behandelt wird.

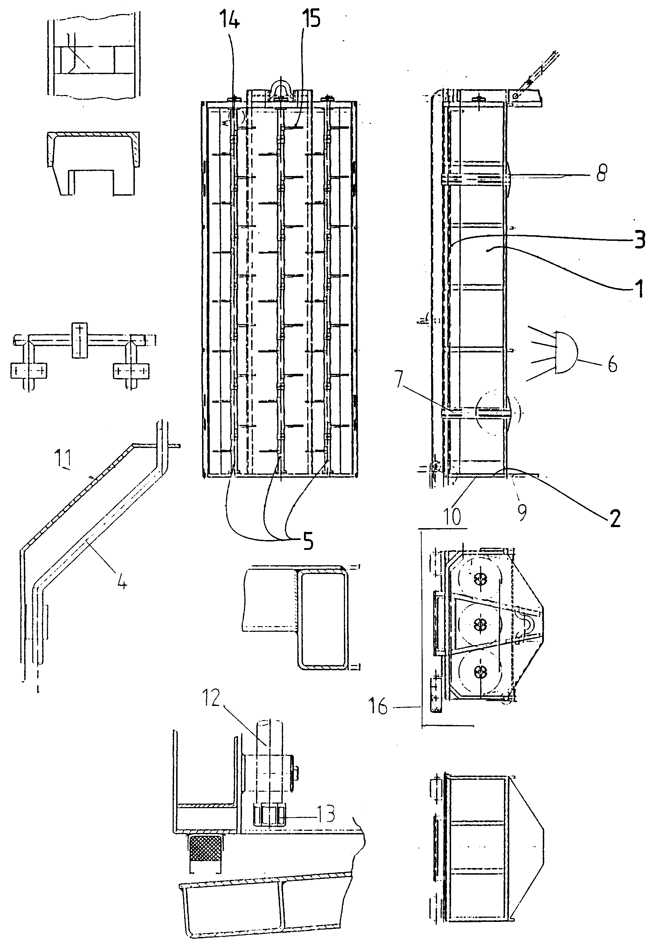

**Patentansprüche**

1.  Flachbett-Reaktor zur biologischen Reinigung von mit gesundheitsschädlichen Stoffen, insbesondere Kohlenwasserstoffen verunreinigten Feststoffen z.B. mit hohem Ton- und Schluffgehalt durch Bakterienkulturen und wässerige Nährlösungen,

    **dadurch gekennzeichnet**,

    daß der Flachbett-Reaktor aus einer nach oben offenen, rechteckigen Wanne (1) mit Klapptür (2) und siebartig gelochtem Boden besteht, sowie mit Belüftungs- und - je nach Bodenart - mit einer optionalen Mischeinrichtung (5) und einem Berieselungssystem (6) ausgerüstet ist.

2.  Flachbett-Reaktor nach Anspruch 1,

    **dadurch gekennzeichnet**,

    daß die Wände der Wanne (1) als stapelbares, kippbares Tragegerüst (7) mit Halterungen (8) ausgestaltet sind.

3.  Flachbett-Reaktor nach Anspruch 1 und 2,

    **dadurch gekennzeichnet**,

    daß mehrere Flachbett-Reaktoren über eine Bodenwanne (16) gestapelt sind.

4. Flachbett-Reaktor nach Anspruch 1 und 2,

   **dadurch gekennzeichnet,**

   daß an den Ecken der Wanne (1) kranfähige Anschlagösen (9) wie z.B. Containeranschlagecken angebracht sind.

5. Flachbett-Reaktor nach Anspruch 1,

   **dadurch gekennzeichnet,**

   daß eine Wand der Wanne (1) als Klapptür (2) mit Dichtung (10) gestaltet ist.

6. Flachbett-Reaktor nach Anspruch 1,

   **dadurch gekennzeichnet,**

   daß die perforierten Belüftungsrohre (12) der Belüftungseinrichtung (4) an den Innenwänden (11) der Wanne und am Boden (1) in Längsrichtung fest angebracht sind.

7. Flachbett-Reaktor nach Anspruch 6,

   **dadurch gekennzeichnet,**

   daß die der Stirnseite gegenüberliegenden Enden der Belüftungsrohre (12) mit Kappen (13) verschlossen sind.

8. Flachbett-Reaktor nach Anspruch 1,

   **dadurch gekennzeichnet,**

   daß die Mischeinrichtungen (5) aus mehreren, vorzugsweise aus drei bis vier achsparallel angeordneten Mischerwellen (14) mit aufgesetzten Rührpaddeln (15) bestehen.

9. Flachbett-Reaktor nach Anspruch 1 und 8,

   **dadurch gekennzeichnet,**

   daß die Mischeinrichtungen (5) mit einem externen hydraulischen oder elektrischen Antrieb ausgestattet sind.

10. Flachbett-Reaktor nach Anspruch 1,

    **dadurch gekennzeichnet,**

    daß der Flachbett-Reaktor mit einem Berieselungssystem (6) ausgerüstet ist.

11. Flachbett-Reaktor nach Anspruch 1,

    **dadurch gekennzeichnet,**

    daß der siebartig gelochte Boden (3) der Wanne (1) mit einer Filterschicht bedeckt ist.

12. Flachbett-Reaktor nach Anspruch 1 bis 11,

    **dadurch gekennzeichnet,**

    daß der Flachbett-Reaktor horizontal und transportabel gestaltet ist.

13. Verfahren zur biologischen Reinigung von Böden mit einem Flachbett-Reaktor nach einem oder mehreren der Ansprüche 1 - 12,

    **dadurch gekennzeichnet,**

    daß die maximale Masse der eingebrachten Feststoffe 28 Mg beträgt und bei einer Betriebstemperatur von 25 ° C und in dem Reaktor - je nach Kontaminationsgrad - für eine Verweilzeit von bis zu mehreren Monaten behandelt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 720 833 (XENEX GESELLSCHAFT ZUR BIOTECHNISCHEN SCHADSTOFFSANIERUNG MBH) <br> * Spalte 8, Zeile 57 - Spalte 9, Zeile 6 * <br> * Spalte 9, Zeile 66 - Spalte 10, Zeile 34 * <br> * Spalte 11, Zeile 41 - Zeile 51 * <br> * Spalte 12, Zeile 30 - Zeile 46 * <br> * Spalte 16, Zeile 53 - Spalte 18, Zeile 29 * <br> * Spalte 25, Zeile 49 - Zeile 66 * <br> * Spalte 26, Zeile 51 - Spalte 28, Zeile 12 * <br> * Abbildungen * | 1,10,11 | B09B3/00 |
| A | | 6,9,12, 13 | |
| A | US-A-4 713 340 (CRAWFORD) <br><br> * Spalte 8, Zeile 17 - Spalte 9, Zeile 9; Abbildung 2 * <br> --- | 1,10,11, 13 | |
| A | US-A-4 962 034 (KHAN) <br><br> * das ganze Dokument * <br> --- | 1,9,10, 13 | |
| A | US-A-4 850 745 (HATER ET AL) <br> * Spalte 3, Zeile 41 - Spalte 4, Zeile 39; Abbildungen * <br> --- | 1,6,7,13 | RECHERCHIERTE SACHGEBIETE (Int. Cl.5) <br><br> B09B <br> B65D |
| A | DE-C-3 722 277 (HUNDENBORN) <br> * Spalte 4, Zeile 28 - Spalte 5, Zeile 17; Abbildungen * <br> --- | 1,8,9,12 | |
| A | DE-A-3 805 375 (ABS-TRANSPORT GMBH) <br> * Spalte 8, Zeile 31 - Zeile 44 * <br> * Spalte 9, Zeile 5 - Zeile 7 * <br> * Spalte 10, Zeile 17 - Zeile 35; Abbildungen * <br> --- | 2,4,5,12 | |
| A | EP-A-0 298 381 (BOCK) <br> * Spalte 3, Zeile 58 - Spalte 4, Zeile 39; Abbildung * <br><br> ----- | 2,12 | |

Der vorliegende Recherchenbericht wurde für alle Patentansprüche erstellt

| Recherchenort | Abschlußdatum der Recherche | Prüfer |
|---|---|---|
| DEN HAAG | 15 MAI 1992 | VAN DER ZEE W.T. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

...............................................................................

& : Mitglied der gleichen Patentfamilie, übereinstimmendes Dokument

EPO FORM 1503 03.82 (P0403)